# EUROPEAN PATENT APPLICATION

(11) **EP 4 442 835 A1**
(43) Date of publication of application: **09.10.2024**
(21) Application number: 22901000.4
(22) Date of filing: 28.10.2022
(51) Int. Cl.: C12Q 1/68, G01N 33/543

(54) **ANALYSIS METHOD**

(30) Priority: 02.12.2021 JP 2021196185
(71) Applicant: DENSO CORPORATION, Kariya-shi, Aichi 448-8661 (JP)
(72) Inventor: HAYAKAWA, Kei, Kariya-shi, Aichi 448-8661 (JP); ASANO, Mana, Kariya-shi, Aichi 448-8661 (JP); NUKAZUKA, Akira, Kariya-shi, Aichi 448-8661 (JP); NAKAGAWA, Kazuhisa, Kariya-shi, Aichi 448-8661 (JP); NIIMOTO, Mai, Kariya-shi, Aichi 448-8661 (JP); KANO, Kazuhiko, Kariya-shi, Aichi 448-8661 (JP)
(74) Representative: Kuhnen & Wacker Patent- und Rechtsanwaltsbüro PartG mbB
(86) International application number: PCT/JP2022/040458
(87) International publication number: WO 2023/100567

(57) **Abstract**

An analysis method for a target substance (7A, 7B, 7C) includes: mixing a conjugate (1A, 1B, 1C) and a sample including the target substance to produce a mixture (21), the conjugate including a labeled substance (5A, 5B, 5C) and a first binding substance (3A, 3B, 3C) having activity to bind to the target substance; bringing the mixture into contact with a second binding substance (19, 19A, 19B, 19C) fixed to a base (12); and detecting a phenomenon caused by the labeled substance bound to the target substance that is bound to the second binding substance. The first binding substance is a substance including a nucleic acid, or a substance including an amino acid. The base is provided in each of regions (17A, 17B, 17C). The target substance to which the second binding substance binds is different among the regions.

## Description

### CROSS REFERENCE TO RELATED APPLICATION

This application is based on Japanese Patent Application No. 2021-196185 filed on December 2, 2021, the disclosure of which is incorporated herein by reference.

### TECHNICAL FIELD

The present disclosure relates to an analysis method.

### BACKGROUND ART

Patent document 1 discloses an analysis method for a target substance by using a conjugate. The conjugate includes a binding substance and a labeled substance. The binding substance has activity to bind to the target substance. The labeled substance induces an observable phenomenon.

In the analysis method for the target substance, a sample containing the target substance is mixed with the conjugate. Fusion bodies, which have not been bound to the target substance, are then removed. Thereafter, the occurrence of the phenomenon caused by conjugates bound to the target substance is detected.

### PRIOR ART LITERATURES

### PATENT LITERATURE

Patent document 1 : JP 2019-33750 A

### SUMMARY OF INVENTION

As a result of the detailed studies conducted by the inventors, the following issues have been found. Samples may contain a plurality of kinds of target substances. In this case, the plurality of kinds of target substances needs to be detected individually.

One aspect of the present disclosure preferably provides an analysis method that can individually detect a plurality of kinds of target substances contained in a sample.

According to an aspect of the present disclosure, an analysis method for a target substance includes: mixing conjugates and a sample including the target substances to produce a mixture, the conjugates including first binding substances, each having activity to bind to the corresponding target substance, and labeled substances; bringing the mixture into contact with second binding substances fixed to a base, each of the second binding substances having activity to bind to the corresponding target substance; removing the target substance not bound to the second binding substance and the conjugate not bound to the target substance that is bound to the second binding substance; and detecting a phenomenon caused by the labeled substance included in the conjugate bound to the target substance that is bound to the second binding substance. The first binding substance is a substance including a nucleic acid, or a substance including an amino acid. The base is provided in each of regions. The target substance to which the second binding substance binds is different for each region.

According to the analysis method of the one aspect of the present disclosure, a plurality of kinds of target substances contained in a sample can be detected individually.

### BRIEF DESCRIPTION OF DRAWINGS

FIG. 1 is an explanatory diagram showing the structure of a plurality of kinds of conjugates and the binding of a conjugate and a target substance.
FIG. 2 is an explanatory diagram showing steps of an analysis method.
FIG. 3 is an explanatory diagram showing the structure of a second binding substance fixed to a granular material.
FIG. 4 is an explanatory diagram showing a mixture obtained by mixing three kinds of fused bodies and a sample.
FIG. 5 is a flowchart showing a method of synthesizing the conjugate.
FIG. 6 is a flowchart showing a method of binding a second binding substance to a plate.
FIG. 7 is an explanatory diagram showing the structure of an avidin plate.
FIG. 8 is a flowchart showing the analysis method for the target substance.
FIG. 9 is an explanatory diagram showing a state in which two kinds of bound bodies are formed in a mixture containing the conjugates and the target substances.
FIG. 10 is an explanatory diagram showing a state in which one kind of bound bodies are formed in a mixture containing the conjugates and the target substances.
FIG. 11 is an explanatory diagram showing a state in which one kind of bound bodies are formed in a mixture containing the conjugates and the target substances.
FIG. 12 is an explanatory diagram showing a state of the bound body in each well.
FIG. 13 is an explanatory diagram showing metabolism using pNPP as a substrate.
FIG. 14 is an explanatory diagram showing the measurement results of absorbance.
FIG. 15 is an explanatory diagram showing steps of an analysis method of another embodiment.

### DESCRIPTION OF EMBODIMENTS

Exemplary embodiments of the present disclosure will be described with reference to the drawings.

### 1. Target substance

A target substance is the substance to be analyzed. Examples of target substances include proteins, sugar, nucleic acids, low molecular weight compounds, lipids, antigens, and viruses. Viruses include, for example, a novel coronavirus SARS-CoV2.

### 2. Fusion body

The analysis method of the present disclosure uses a conjugate. The conjugate includes a first binding substance and a labeled substance. The first binding substance has activity to bind to the target substance. The first binding substance has an activity, for example, to differentially bind to a specific target substance and is difficult to bind to other substances. Examples of the first binding substance include a substance composed of a nucleic acid, a substance composed of an amino acid, and the like. Examples of the substance composed of a nucleic acid include a nucleic acid aptamer, and the like. Examples of a material composed of an amino acid include a low molecular weight protein preparation and the like. For example, the conjugate can bind to the target substance by including the first binding substance.

The labeled substance induces an observable phenomenon. The observable phenomena include, for example, production, consumption, and absorption of hydrogen ions, potassium ions, sodium ions, calcium ions, lithium ions, ammonium ions, and chloride ions. Observable phenomena include, for example, coloration, fluorescence, luminescence, phosphorescence, endotherm, exotherm, oxidation-reduction, precipitation, and the like.

The labeled substance induces, for example, the production, consumption, or absorption of ions. The labeled substance that induces the production, consumption, or absorption of ions is, for example, an enzyme. The labeled substance inducing the production, consumption, or absorption of ions includes, for example, at least one of 1,3-propanediol dehydrogenase, 15-hydroxyprostaglandin- dehydrogenase, 1H-pyrrole-2-carbonyl-[peptidyl-carrier protein] chlorinase, 2,4-dichlorobenzoyl-CoA reductase, 2,5-dioxovalerate dehydrogenase, 2-aminobenzenesulfonate 2,3-dioxygenase, 2-iminobutanoate/2-iminopropanoate deaminase, 2-enoic acid reductase, 2'-dehydrokanamycin reductase, 3,4-dihydroxyphenylalanine reductive deaminase, 3α-hydroxysteroid 3-dehydrogenase, 3-aminobutyryl-CoA ammonia-lyase, 3-oxo-5α-steroid 4-dehydrogenase, 3-oxosteroid -1 dehydrogenase, 3-chloro-D-alanine dehydrogenase, 4-chlorophenylacetate 3,4-dioxygenase, 4-chlorobenzoyl-CoA dehalogenase, 4-chlorobenzoate dehalogenase, 4-trimethylammoniobutyraldehyde dehydrogenase, 4-methylaminobutanoate oxidase, 4-methylglutamate-ammonia ligase, 5-phosphooxy-L-lysine phosphorylase, 7, 8-didemethyl-8-hydroxy-5-deazariboflavin synthase, 7-carboxy-7-deazaguanine synthase, 7-chloro-L-tryptophan 6-halogenase, 7-cyano-7-deazaguanine-synthase, AMP deaminase, CTP synthase, DDT-dehydrochlorinase, dTDP-4-amino-4,6-dideoxy-D-glucose-ammonia lyase, D-arabinose-1-dehydrogenase, D-arginine dehydrogenase, D-xylose reductase, D-glucosamine-6-phosphate ammonia lyase, D-serine ammonia lyase, D-lactate dehydrogenase, GDP-4-dehydro-6-deoxy-α-D-mannose 3-dehydratase, GMP synthase, L-2-amino-4-chloropent-4-enoate dehydrochlorinase, L-cystine β-lyase, L-cysteine desulphidase, L-cysteate Sulfolyase, L-serine ammonia lyase, L-tryptophan ammonia-lyase, L-lysine cyclodeaminase, N1-acetylpolyamine oxidase, N8-acetylspermidine oxidase, NAD+-diphthamide ADP-ribosyltransferase, NAD+ synthase, NAD +-dinitrogen reductase ADP-D-ribosyltransferase, N-succinlyarginine dihydrolase, S-(hydroxymethyl)mycothiol dehydrogenase, S-carboxymethylcysteine synthase, UDP-2-acetamide-2,6-β-L-arabino-hexyl-4-ose reductase, UDP-N-acetyl-2-amino-2-deoxyglucuronate dehydrogenase, UDP-N-acetyl-D-mannosamine dehydrogenase, UDP-N-acetyl-α-D-quinovosamine dehydrogenase, UDP-N-acetylglucosamine 3-dehydrogenase, UDP-N-acetylglucosamine 6-dehydrogenase, UDP-glucuronate dehydrogenase, β-alanyl-CoA ammonia-lyase, β-ureidopropionase, β-lactamase, γ-butyrobetaine dioxygenase, asparaginyl-tRNA synthetase, asparagine synthetase, aspartate ammonia-lyase, aspartate-ammonia ligase, aspartate semialdehyde dehydrogenase, aspartate dehydrogenase, adenylylsulfate-ammonia adenylyltransferase, adenosyl-chloride synthase, atrazine chlorohydrolase, aminomethyltransferase, alanine dehydrogenase, allantoate deiminase, alkaline phosphatase, alcohol dehydrogenase, aldehyde dehydrogenase, ammonia kinase, ammonia monooxygenase, isocitrate dehydrogenase, imidazole glycerol-phosphate synthase, uricase, urease, ureidoglycolate amidohydrolase, uronate dehydrogenase, ethanolamine ammonia-lyase, erythro-3-hydroxy-L-aspartate ammonia-lyase, octopamine dehydratase, ornithine cyclodeaminase, galactose dehydrogenase, carbamate kinase, carbamoyl-serine ammonia-lyase, carbamoyl phosphate synthetase, carbonyl reductase, formate dehydrogenase, glycine reductase, glycerol-3 phosphate dehydrogenase, glucose-6 phosphate dehydrogenase, glucose oxidase, glucose dehydrogenase, glucokinase, glucosaminate ammonia-lyase, glucosamine-6-phosphate deaminase, glutaminyl-tRNA synthetase, glutamine synthetase, glutamate synthase, glutamate dehydrogenase, crotonoyl-CoA hydratase (crotonoylbenzol-CoA hydratase), chloride peroxidase, coproporphyrinogen dehydrogenase, choline oxidase, choline monooxygenase, cholesterol oxidase, diacetyl reductase, diaminopimelate dehydrogenase, diaminopropionate ammonia lyase, shikimate dehydrogenase, cyclohexane-1,2-diol dehydrogenase, dichlorochromopyrrolate synthase, dichloromethane dehalogenase, cystathionine γ-lyase, cysteine-S-conjugate β-lyase, dihydrolipoyl dehydrogenase, diphthine-ammonia ligase, cinnamoyl-coenzyme A-reductase, succinylglutamate-semialdehyde dehydrogenase, stachydrine N-demethylase, threo-3-hydroxy-D-aspartate ammonia lyase, threo-3-hydroxy-L-aspartate ammonia lyase, threonine ammonia-lyase, serine sulfate ammonia lyase, thioredoxin disulfide reductase, tyrosine ammonia lyase, tyrosine phenol lyase, tetrachloroethene-reducing dehalogenase, tetracycline 7-halogenase, tryptophanase, tryptophan 5-halogenase, tryptophan 6-halogenase, tryptophan 7-halogenase, nicotinamide adenine dinucleotide phosphate-dehydrogenase, nicotinamide adenine dinucleotide phosphate-heme protein reductase, nitrogenase, vanadium dependent nitrogenase, hapalindole-type alkaloid chlorinase, histidine ammonia-lyase, hydrazine synthase, hydrazine dehydrogenase, hydroxymethylbilane synthase, hydroxylamine reductase, pyridoxal 5'-phosphate synthase, arsenate reductase, phenylalanine/tyrosine ammonia lyase, phenylalanine 2-monooxygenase, phenylalanine ammonia-lyase, ferredoxin-nicotinamide adenine dinucleotide phosphate reductase, ferredoxin-nitrite reductase, fumarate reductase, protoporphyrinogen oxidase, betaine-aldehyde dehydrogenase, betaine reductase, peroxidase, homocysteine desulfhydrase, homospermidine synthase, form im idoyltetrahydrofolate cyclodeaminase, myeloperoxidase, myeloperoxidase, methanol dehydrogenase, methane monooxygenase, methionine γ-lyase, methylaspartate ammonia-lyase, methylamine dehydrogenase, methylenediurea deaminase, lactaldehyde dehydrogenase, ribose -5 phosphate-ammonia ligase, malate dehydrogenase, rubredoxin-nicotinamide adenine dinucleotide phosphate reductase, chlorite O2-lyase, nitrite reductase, sulfite dehydrogenase, chloride peroxidase, chlorate reductase, lipid II isoglutaminyl synthase, carbonic anhydrase, and nonspecific polyamine oxidase.

The labeled substance has, for example, at least one of an enzyme, a DNA zyme, an RNA zyme, magnetic labeling, fluorescent labeling, a chemiluminescent probe, or nanoparticles.

Each of the first binding substance and the labeled substance includes, for example, a chemical substituent or a nucleic acid binding protein. For example, a labeled body is fused to the binding substance by binding the chemical substituent or nucleic acid binding protein included in the labeled body to the chemical substituent or nucleic acid binding protein included in the binding substance.

The chemical substituent included in the first binding substance has, for example, at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, and 5-halouracil. Examples of the nucleic acid binding protein included in the first binding substance include a zinc finger, CRISPR, and the like. Examples of 5-halouracil include 5-iodouracil and 5-bromouracil. 5-Halouracil is a chemical substituent that enables UV crosslinking.

The chemical substituent included in the labeled substance has, for example, at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, and 5-halouracil. Examples of the nucleic acid binding protein included in the labelled substance include a zinc finger, CRISPR, and the like. Examples of 5-halouracil include 5-iodouracil and 5-bromouracil. 5-Halouracil is a chemical substituent that enables UV crosslinking.

The chemical substituent included in the labeled substance is different from the chemical substituent included in the first binding substance. For example, when the chemical substituent included in the labeled substance is biotin, the chemical substituent included in the first binding substance is avidin, streptavidin, or neutravidin.

The analysis method of the present disclosure uses, for example, a plurality of kinds of conjugates. The target substances that bind to the conjugates are different for each type of conjugate.

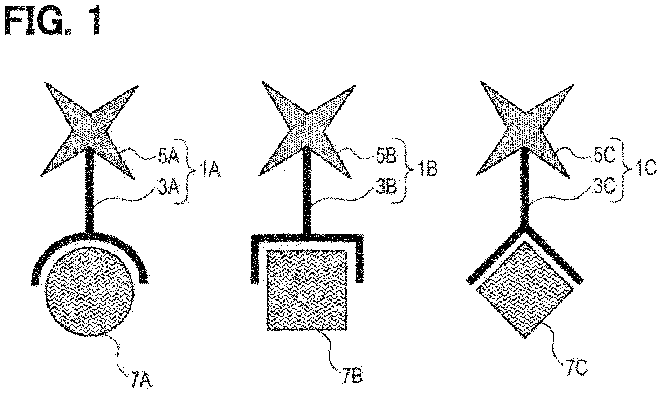

In the analysis method of the present disclosure, for example, a plurality of kinds of conjugates 1A, 1B, and 1C shown in FIG. 1 are used. The conjugate 1A includes a first binding substance 3A and a labeled substance 5A. The conjugate 1B includes a first binding substance 3B and a labeled substance 5B. The conjugate 1C includes a first binding substance 3C and a labeled substance 5C.

The conjugate 1A binds to the target substance 7A. More specifically, the first binding substance 3A included in the conjugate 1A binds to the target substance 7A. The conjugate 1A does not bind to the target substances 7B and 7C described below.

The conjugate 1B binds to the target substance 7B. More specifically, the first binding substance 3B included in the conjugate 1B binds to the target substance 7B. The conjugate 1B binds neither to the target substance 7A nor to target substance 7C to be mentioned later.

The conjugate 1C binds to the target substance 7C. More specifically, the first binding substance 3C included in the conjugate 1C binds to the target substance 7C. The conjugate 1C does not bind to the target substance 7A and 7B. The target substances 7A, 7B, and 7C are different substances.

The labeled substances 5A, 5B, and 5C may be the same or may be different from each other. The observable phenomena caused by the labeled substances 5A, 5B, and 5C may be the same or different from each other.

### 3. Base and second binding substance

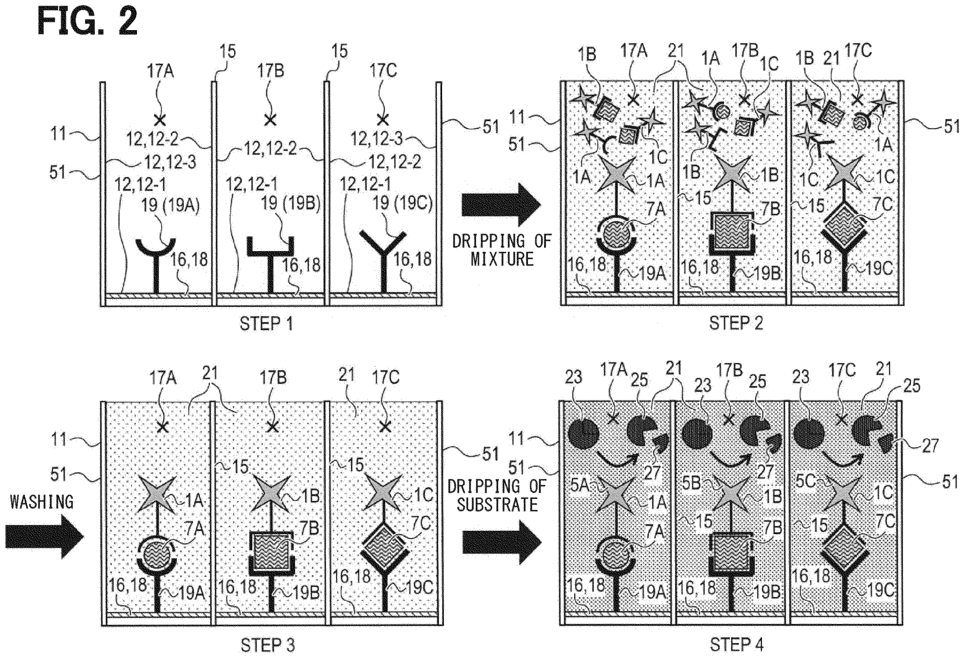

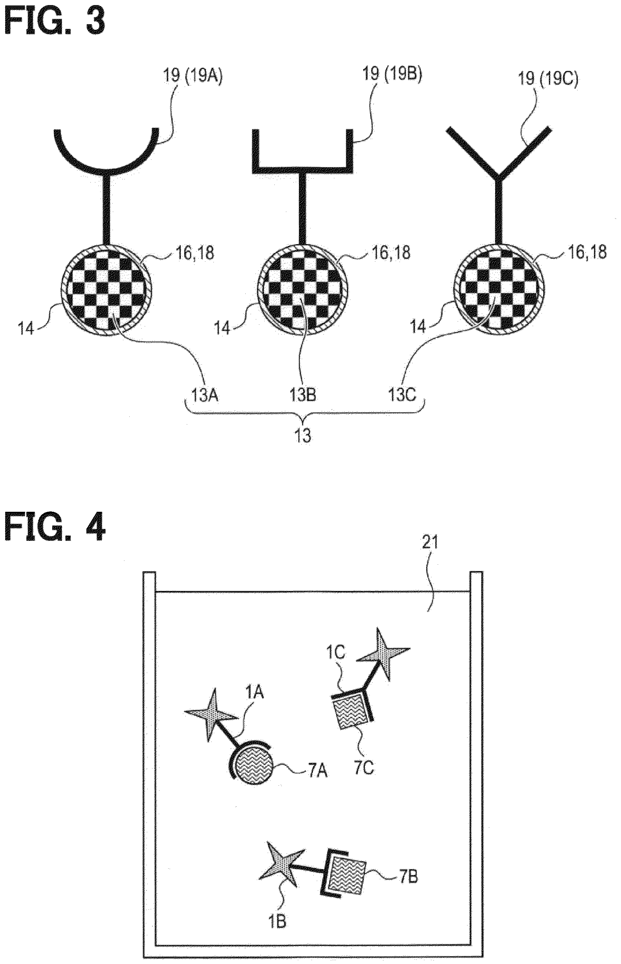

The analysis method of the present disclosure uses a base and the second binding substance. The base is, for example, an inner surface 12 of a container 11 shown in FIG. 2, a surface 14 of a granular material 13 shown in FIG. 3, or the like. The granular material 13 includes, for example, magnetic beads and the like.

The container 11 has an outer wall 51. The outer wall 51 is a wall separating the interior of the container 11 from the exterior thereof. The container 11 has a plurality of partition walls 15 and a plurality of wells 17A, 17B, and 17C inside the outer wall 51. The plurality of wells 17A, 17B, and 17C is separated by the partition walls 15.

The inner surface 12 of the container 11 is present in each of the wells 17A, 17B, and 17C. The inner surface 12 of each of the wells 17A and 17C is composed of a bottom surface 12-1, a first side surface 12-2, and a second side surface 12-3. The inner surface 12 of the well 17B is composed of the bottom surface 12-1 and the first side surface 12-2. The first side surface 12-2 is a surface of the partition wall 15. The second side surface 12-3 is an inner surface of the outer wall 51. The base includes, for example, one or more of the bottom surface 12-1, the first side surface 12-2, or the second side surface 12-3.

When the inner surface 12 of the container 11 is the base, at least a portion of the inner surface 12 includes the chemical substituent 16 or nucleic acid binding protein 18, for example. In the example shown in FIG. 2, the bottom surface 12-1 is the base, and the bottom surface 12-1 includes the chemical substituent 16 or nucleic acid binding protein 18. When the surface 14 of the granular material 13 is the base, the surface 14 includes, for example, the chemical substituent 16 or nucleic acid binding protein 18.

The base is provided for each of the plurality of regions. For example, as shown in FIG. 2, each of the plurality of wells 17A, 17B, and 17C included in the container 11 corresponds to one region. Each of the plurality of wells 17A, 17B, and 17C is provided with the inner surface 12. The wells 17A, 17B, and 17C are regions constituted of the inner surfaces 12 of the container 11. The bottom surface 12-1 provided in each of the wells 17A, 17B, and 17C corresponds to the base. The bottom surface 12-1, which is the base, includes the chemical substituent 16 or nucleic acid binding protein 18.

For example, as shown in FIG. 3, the granular material 13 is classified into one or more granular materials 13A, one or more granular materials 13B, and one or more granular materials 13C. For example, the granular materials 13A, 13B, and 13C are arranged in separate regions. For example, the granular material 13A is housed in the well 17A of the container 11, the granular material 13B is housed in the well 17B of the container 11, and the granular material 13C is housed in the well 17C of the container 11. Each of the wells 17A, 17B, and 17C is one region. The granular materials 13A, 13B, and 13C are, for example, magnetic. For example, the granular materials 13A, 13B, and 13C are held in the corresponding regions by magnetic forces.

For example, the surface 14 of the granular material 13A corresponds to the base provided in the well 17A. The surface 14 of the granular material 13B corresponds to the base provided in the well 17B. The surface 14 of the granular material 13C corresponds to the base provided in the well 17C.

The second binding substance is fixed to the base. The second binding substance has activity to bind to the target substance. The second binding substance is, for example, a protein, sugar, nucleic acid, low molecular weight compound, or lipid.

The second binding substance is fixed to the base in each of the plurality of regions. In the example shown in FIG. 2, the second binding substance 19 is fixed to the bottom surface 12-1 in each of wells 17A, 17B, and 17C. The bottom surface 12-1 corresponds to the base.

As shown in FIG. 3, when the base is the surface 14 of the granular material 13, the second binding substance 19 is fixed to the surface 14 of the granular material 13A, the surface 14 of the granular material 13B, and the surface 14 of the granular material 13C.

The target substance to which the second binding substance binds is different for each region. In the example shown in FIG. 2, the second binding substance 19 fixed to the bottom surface 12-1 of the well 17A is the second binding substance 19A. The second binding substance 19A binds to the target substance 7A. The second binding substance 19A does not bind to the target substance 7B and 7C.

The second binding substance 19 fixed to the bottom surface 12-1 of the well 17B is the second binding substance 19B. The second binding substance 19B binds to the target substance 7B. The second binding substance 19B does not bind to the target substance 7A and 7C.

The second binding substance 19 fixed to the bottom surface 12-1 of the well 17C is the second binding substance 19C. The second binding substance 19C binds to the target substance 7C. The second binding substance 19C does not bind to the target substance 7B and 7A.

Therefore, the target substances 7 to which the second binding substance 19 binds are the target substance 7A in the well 17A, the target substance 7B in the well 17B, and the target substance 7C in the well 17C. That is, the target substance 7 to which the second binding substance 19 binds is different for each region.

Also, in the example shown in FIG. 3, the target substance to which the second binding substance 19 binds is different for each region. The granular materials 13A, 13B, and 13C are arranged in separate regions. The second binding substance 19 fixed to the surface 14 of the granular material 13A is the second binding substance 19A. The second binding substance 19A binds to the target substance 7A. The second binding substance 19A does not bind to the target substance 7B and 7C.

The second binding substance 19 fixed to the surface 14 of the granular material 13B is the second binding substance 19B. The second binding substance 19B binds to the target substance 7B. The second binding substance 19B does not bind to the target substance 7A and 7C.

The second binding substance 19 fixed to the surface 14 of the granular material 13C is the second binding substance 19C. The second binding substance 19C binds to the target substance 7C. The second binding substance 19C does not bind to the target substance 7B and 7A.

Therefore, the target substances 7 to which the second binding substance 19 binds are the target substance 7A in the granular material 13A, the target substance 7B in the granular material 13B, and the target substance 7C in the granular material 13C. That is, the target substance 7 to which the second binding substance 19 binds is different for each region.

Each of the second binding substance 19 and the base includes, for example, a chemical substituent or nucleic acid binding protein. For example, the second binding substance is fixed to the base by binding the chemical substituent or nucleic acid binding protein included in the second binding substance 19 to the chemical substituent 16 or nucleic acid binding protein 18 included in the base.

The chemical substituent included in the second binding substance 19 has, for example, at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, and 5-halouracil. Examples of the nucleic acid binding protein included in the second binding substance 19 include a zinc finger, CRISPR, and the like. Examples of 5-halouracil include 5-iodouracil and 5-bromouracil. 5-Halouracil is a chemical substituent that enables UV crosslinking.

The chemical substituent 16 included in the base has, for example, at least one of biotin, primary amine, azide, alkyne, dibenzocyclooctyne, bicyclononyne, 2 -O-propargyl, 2'-O-propargyl, thiol, avidin, streptavidin, neutravidin, N-hydroxysuccinimide, maleimide, and 5-halouracil. Examples of the nucleic acid binding protein 18 included in the base include a zinc finger, CRISPR, and the like. Examples of 5-halouracil include 5-iodouracil and 5-bromouracil. 5-Halouracil is a chemical substituent that enables UV crosslinking.

The chemical substituent included in the second binding substance 19 is different from the chemical substituent 16 included in the base. For example, when the chemical substituent included in the second binding substance 19 is biotin, the chemical substituent 16 included in the base is avidin, streptavidin, or neutravidin.

### 4. Analysis method

In the analysis method of the present disclosure, the conjugate and a sample containing the target substance are mixed together to produce a mixture. The mixture includes, for example, a plurality of kinds of conjugates. In the mixture, at least a portion of the conjugate bind to the target substance. More specifically, the first binding substance included in the conjugate binds to the target substance.

In a case where the mixture contains a plurality of kinds of conjugates, each type of conjugate binds to a corresponding target substance if the corresponding target substance is present in the mixture. The corresponding target substance is a target substance having activity to cause the conjugate to bind to the target substance.

For example, in the example shown in FIG. 4, three kinds of conjugates 1A, 1B, and 1C are mixed with a sample containing target substances 7A, 7B, and 7C to produce a mixture 21. When the target substance 7A is present in the sample, the conjugate 1A binds to the target substance 7A. The conjugates 1B and 1C do not bind to the target substance 7A.

When the target substance 7B is present in the sample, the conjugate 1B binds to the target substance 7B. The conjugates 1A and 1C do not bind to the target substance 7B. When the target substance 7C is present in the sample, the conjugate 1C binds to the target substance 7C. The conjugates 1A and 1B do not bind to the target substance 7C.

Next, in the analysis method of the present disclosure, the second binding substance 19 fixed to the base is brought into contact with the mixture 21. At this time, the second binding substance 19 and the target substance contained in the mixture 21 are bound to each other.

The base is provided for each of the plurality of regions. The target substance to which the second binding substance 19 binds is different for each region. Therefore, the target substance to which the second binding substance 19 binds is different for each region.

In the example shown in FIG. 2, the mixture 21 is placed in each of the wells 17A, 17B, and 17C as shown in STEPS 1 and 2. Each of the wells 17A, 17B, and 17C is one region. The mixture 21 placed in the well 17A, the mixture 21 placed in the well 17B, and the mixture 21 placed in the well 17C are separated, for example, by the partition walls 15, and thus they do not mix with each other.

After the mixture 21 is added, the second binding substance 19A, which is fixed to the bottom surface 12-1, comes into contact with the mixture 21 in the well 17A. In a case where the target substance 7A is present in the mixture 21, the target substance 7A binds to the second binding substance 19A. The conjugate 1A binds to the target substance 7A. Therefore, when the target substance 7A is present in the mixture 21, as shown in STEP 2 of FIG. 2, the conjugate 1A binds to the target substance 7A, and thus the target substance 7A binds to the second binding substance 19A in the well 17A. As a result, the conjugate 1A is fixed to the second binding substance 19A via the target substance 7A.

In the well 17B, the second binding substance 19B, which is fixed to the bottom surface 12-1, comes into contact with the mixture 21. In a case where the target substance 7B is present in the mixture 21, the target substance 7B binds to the second binding substance 19B. The conjugate 1B binds to the target substance 7B. Therefore, when the target substance 7B is present in the mixture 21, as shown in STEP 2 of FIG. 2, the conjugate 1B binds to the target substance 7B, and thus the target substance 7B binds to the second binding substance 19B in the well 17B. As a result, the conjugate 1B is fixed to the second binding substance 19B via the target substance 7B.

In the well 17C, the second binding substance 19C, which is fixed to the bottom surface 12-1, comes into contact with the mixture 21. In a case where the target substance 7C is present in the mixture 21, the target substance 7C binds to the second binding substance 19C. The conjugate 1C binds to the target substance 7C. Therefore, when the target substance 7C is present in the mixture 21, as shown in STEP 2 of FIG. 2, the conjugate 1C binds to the target substance 7C, and thus the target substance 7C binds to the second binding substance 19C in the well 17C. As a result, the conjugate 1C is fixed to the second binding substance 19C via the target substance 7C.

Next, the analysis method of the present disclosure removes, from the mixture 21, the target substance not bound to the second binding substance 19 fixed to the bottom surface 12-1, and the conjugate not bound to the target substance that is bound to the second binding substance 19 fixed to the bottom surface 12-1. Methods of the removal include, for example, washing.

In the example shown in FIG. 2, as shown in STEP 2 and 3, target substances 7A, 7B, and 7C not bound to the second binding substance 19A that is fixed to the bottom surface 12-1, and conjugates 1A, 1B, and 1C not bound to the target substance 7A that is bound to the second binding substance 19A fixed to the bottom surface 12-1 are removed from the mixture 21 in the well 17A.

Further, in the well 17B, the target substances 7A, 7B, and 7C not bound to the second binding substance 19B fixed to the bottom surface 12-1, as well as the conjugates 1A, 1B, and 1C not bound to the target substance 7B that is bound to the second binding substance 19B fixed to the bottom surface 12-1 are removed from the mixture 21.

Further, in the well 17C, the target substances 7A, 7B, and 7C not bound to the second binding substance 19C fixed to the bottom surface 12-1, as well as the conjugates 1A, 1B, and 1C not bound to the target substance 7C that is bound to the second binding substance 19C fixed to the bottom surface 12-1 are removed from the mixture 21.

Next, the analysis method of the present disclosure detects a phenomenon caused by a labeled substance included in the conjugate that is bound to the target substance bound to the second binding substance 19. For example, in each of a plurality of regions, the phenomenon caused by the labeled substance included in the conjugate is detected.

In the example shown in FIG. 2, as shown in STEP 4, a process of detecting the phenomenon caused by the labeled substance 5A included in the conjugate 1A (hereinafter referred to as a phenomenon A) is performed in the well 17A.

In a case where the sample contains the target substance 7A, the phenomenon A can be detected. The phenomenon A is caused by the conjugate 1A bound to the target substance 7A that is bound to the second binding substance 19A. In a case where the sample does not contain the target substance 7A, the phenomenon A cannot be detected.

Thus, when the phenomenon A is detected in the well 17A, it can be judged that the sample contains the target substance 7A. The amount and concentration of the target substance 7A can be estimated by the extent of the phenomenon A.

A process of detecting the phenomenon caused by the labeled substance 5B included in the conjugate 1B (hereinafter referred to as a phenomenon B) is performed in the well 17B. In a case where the sample contains the target substance 7B, the phenomenon B can be detected. The phenomenon B is caused by the conjugate 1B bound to the target substance 7B that is bound to the second binding substance 19B. In a case where the sample does not contain the target substance 7B, the phenomenon B cannot be detected.

Thus, when the phenomenon B is detected in the well 17B, it can be judged that the sample contains the target substance 7B. The amount and concentration of the target substance 7B can be estimated by the extent of the phenomenon B.

A process of detecting the phenomenon caused by the labeled substance 5C included in the conjugate 1C (hereinafter referred to as a phenomenon C) is performed in the well 17C. In a case where the sample contains the target substance 7C, the phenomenon C can be detected. The phenomenon C is caused by the conjugate 1C bound to the target substance 7C that is bound to the second binding substance 19C. In a case where the sample does not contain the target substance 7C, the phenomenon C cannot be detected.

Thus, when the phenomenon C is detected in the well 17C, it can be judged that the sample contains the target substance 7C. The amount and concentration of the target substance 7C can be estimated by the extent of the phenomenon C.

Since the regions where the phenomena A, B, and C are caused are different from each other, the user can distinguish among the phenomena A, B, and C.

For example, in a case where the phenomenon is the production, consumption, or absorption of hydrogen ions, potassium ions, sodium ions, calcium ions, lithium ions, ammonium ions, or chloride ions, the phenomenon can be detected using, for example, a pH meter or ion-sensitive field effect transistor.

For example, in a case where the phenomenon is coloration, luminescence, fluorescence, or phosphorescence, the phenomenon can be detected using, for example, a light receiving device.

For example, in a case where the phenomenon is endotherm or exotherm, the phenomenon can be detected using, for example, a thermal analyzer.

For example, in a case where the phenomenon is oxidation-reduction, the phenomenon can be detected using, for example, a potentiometer or ion-sensitive field effect transistor.

For example, in a case where the phenomenon is precipitation, the phenomenon can be detected using, for example, a mass spectrometer, absorptiometer, or spectrophotometer.

The labeled substances 5A, 5B, and 5C are, for example, enzymes. The phenomena A, B, and C are phenomena of the occurrence of the production, consumption, or absorption of ions that is caused by products 25 and 27 generated from the substrate 23 due to the action of the enzymes. The substrate 23 is mixed with the mixture 21, for example, between STEP 3 and STEP 4.

### 5. Effects exhibited by analysis method

(1A) According to the analysis method of the present disclosure, a plurality of target substances contained in a sample can be detected individually. In the examples shown in FIGS. 1 to 3, the mixture 21 is placed in each of the wells 17A, 17B, and 17C, for example. In the well 17A, the phenomenon A is detected. In the well 17B, the phenomenon B is detected. In the well 17C, the phenomenon C is detected. As a result, the target substances 7A, 7B, and 7C contained in the sample can be detected individually. In particular, according to the analysis method of the present disclosure, the target substances contained in the sample can be detected individually.

(1B) The base is, for example, the inner surface 12 of the container 11 or the surface 14 of the granular material 13. The second binding substance 19 is fixed to, for example, the inner surface 12 of the container 11 or the surface 14 of the granular material 13. After the mixture 21 is placed in the wells 17A, 17B, and 17C, the second binding substance 19 binds to the target substance 7, and the target substance 7 binds to the conjugate 1. When the base is the inner surface 12 of the container 11, the second binding substance 19 is fixed to the container 11, so that the target substance 7 bound to the second binding substance 19 and the conjugate 1 bound to the target substance 7 are allowed to remain in the wells 17A, 17B, and 17C, while other substances contained in the mixture 21 can be easily removed and washed. When the base is the surface 14 of the granular material 13, for example, if the granular material 13 is composed of magnetic beads, the magnetic beads are allowed to remain within the wells 17A, 17B, and 17C using a magnet, so that other substances contained in the mixture 21 can be easily removed and washed.

(1C) The second binding substance 19 is, for example, a protein, sugar, nucleic acid, low molecular weight compound, or lipid. According to the analysis method of the present disclosure, various target substances 7 can be detected depending on the type of the second binding substance 19.

(1D) The labeled substances 5A, 5B, and 5C are, for example, at least one of enzymes, DNA zymes, RNA zymes, magnetic labels, fluorescent labels, chemiluminescent probes, and nanoparticles. According to the analysis method of the present disclosure, a wide variety of phenomena can be handled, and the detection methods for the phenomena are not limited.

(1E) Examples of the observable phenomena caused by the conjugates 1A, 1B, and 1C bound to the target substances 7A, 7B, and 7C, respectively, include production, consumption, and absorption of hydrogen ions, potassium ions, sodium ions, calcium ions, lithium ions, ammonium ions, and chloride ions, as well as coloration, fluorescence, luminescence, phosphorescence, endotherm, exotherm, oxidation-reduction, and precipitation. According to the analysis method of the present disclosure, a wide variety of phenomena can be handled, and the detection methods for the phenomena are not limited.

### 6. Example

### (6-1) Synthesis of conjugates 1A and 1C

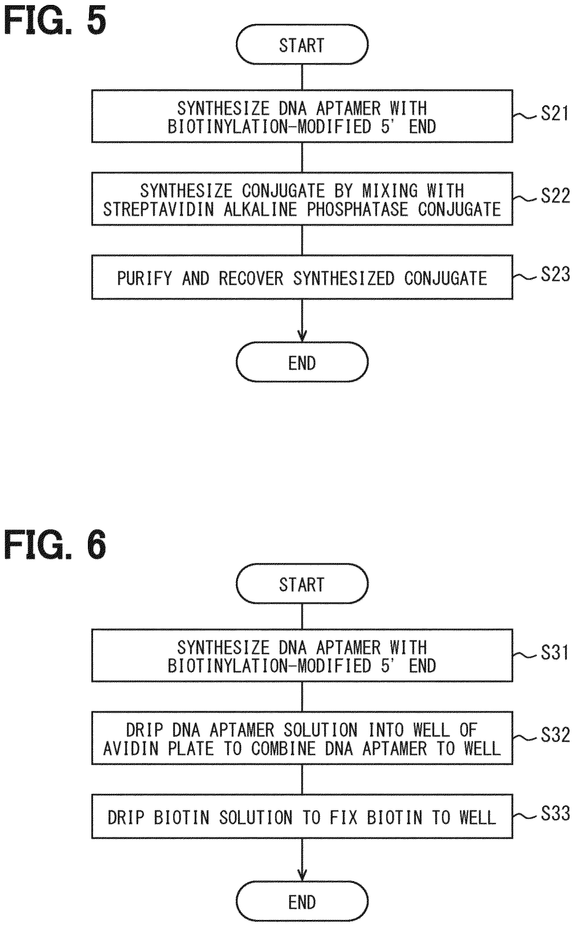

The conjugates 1A and 1C were synthesized by the method shown in FIG. 5. First, in the process of S21 in FIG. 5, each of the first binding substances 3A and 3C was chemically synthesized by an in-vitro process. The first binding substance 3A was a DNA aptamer having a base sequence of SEQ ID NO: 1 with a biotinylation-modified 5' end. The first binding substance 3C was a DNA aptamer having a base sequence of SEQ ID NO: 2 with a biotinylation-modified 5' end.

The base sequence of the first binding substance 3A was "5'-CAGCACCGAC CTTGTGCTTT GGGAGTGCTG GTCCAAGGGC GTTAATGGAC A-3'". The first binding substance 3A is described in Anal. Chem. 2020, 92, 9895-9900 (hereafter cited as Reference 1).

According to the description in Reference 1, the first binding substance 3A is a DNA aptamer whose target substance is an RBD in a spike protein of the novel coronavirus SARS-CoV-2.

The base sequence of the first binding substance 3C was "5'-GCTGGATGTCGCTTACGACAATATTCCTTAGGGGCACCGCTACATTGACACATC CAGC-3' ". The base sequence of the first binding substance 3C is described in Chem. Commun. 2020, 56, 10235-10238 (hereinafter referred to as Reference 2).

According to the description in Reference 2, the first binding substance 3C is a DNA aptamer whose target material is a nucleocapsid protein of the novel coronavirus SARS-CoV-2.

The first binding substance 3A was dissolved in a phosphate buffer solution (hereinafter referred to as 1x PBS/T) to produce a first binding substance 3A solution. The first binding substance 3C was also dissolved in 1x PBS/T to produce a first binding substance 3C solution.

Each of the concentrations of the first binding substance 3A in the first binding substance 3A solution and the first binding substance 3C in the first binding substance 3C solution was 10 µmol/l.

1xPBS/T was a phosphate buffer solution obtained by diluting 10x PBS/T (FUJIFILM Wako Pure Chemical Corporation, product number MB-075-1000) with 10 parts of ultrapure water. The first binding substance 3A solution and the first binding substance 3C solution were heated at 95°C and then slowly cooled.

Next, in the process of S22 in FIG. 5, the labeled substance 5 was prepared. The labeled substance 5 was a streptavidin alkaline phosphatase conjugate (Thermo Fisher Scientific Inc., product number S921). The labeled substance 5 was an enzyme. The streptavidin alkaline phosphatase conjugate is obtained by modifying alkaline phosphatase with streptavidin. Streptavidin is a chemical substituent that modifies alkaline phosphatase. The labeled substance 5 was dissolved in 1x PBS/T to produce a labeled substance solution. The concentration of the labeled substance in the labeled substance solution was 20 µmol/l.

Then, 100 µL of the first binding substance 3A solution was mixed with 5 µL of the labeled material solution and allowed to stand for 1 hour at room temperature. At this time, the first binding substance 3A and the labeled material 5 were fused together by the biotin-streptavidin interaction to thereby synthesize the conjugate 1A.

Also, 100 µL of the first binding substance 3C solution was mixed with 5 µL of the labeled substance solution and allowed to stand for 1 hour at room temperature. At this time, the first binding substance 3C and the labeled material 5 were fused together by the biotin-streptavidin interaction to thereby synthesize the conjugate 1C.

Next, in the process of S23 in FIG. 5, the conjugates 1A and 1C were purified and recovered. Specifically, the first binding substance 3A, which was not fused with the labeled substance 5, was separated from the conjugate 1A using an ultrafiltration filter. Then, the conjugate 1A was recovered by dissolving the conjugate 1A in the 1x PBS/T.

The first binding substance 3C, which was not fused with the labeled substance 5, was separated from the conjugate 1C using the ultrafiltration filter. Then, the conjugate 1C was recovered by dissolving the conjugate 1C in the 1x PBS/T.

Next, a conjugate 1A solution containing the conjugate 1A and a conjugate 1C solution containing the conjugate 1C were produced individually. Each of the concentrations of the labeled substance 5 in the conjugate 1A solution and the labeled substance 5 in the conjugate 1C solution was 20 µmol/l.

The first binding substances 3A and 3C may be binding substances other than DNA aptamers. The first binding substances 3A and 3C may be, for example, a low molecular weight protein preparation or an RNA aptamer. Examples of low molecule weight protein preparations include fragment antibodies, single chain antibodies, diabodies, nanobodies, VHHs, peptide aptamers, and the like.

The base sequences of the DNA aptamers constituting the first binding substances 3A and 3C may be base sequences other than those of SEQ ID NO: 1 and SEQ ID NO: 2. The base sequence of the DNA aptamer can be selected according to the target substance. The fusion of the first binding substances 3A and 3C with the labeled substance 5 may be a fusion other than a fusion based on the biotin-streptavidin interaction. When nucleic acid aptamers are used as the first binding substances 3A and 3C, ends other than the 5' end in the nucleic acid aptamer may be fused with the labeled substance 5.

When low molecule protein preparations are used as the first binding substances 3A and 3C, the N-terminus in the low molecule protein preparation may be fused with the labeled substance 5, or the C-terminus may be fused with the labeled substance 5.

### (6-2) Binding of second binding substances 19A and 19C to plate

The second binding substances 19A and 19C were adhered to the wells in the way shown in FIG. 6. First, in the process of S31 in FIG. 6, the second binding substances 19A and 19C were chemically synthesized by an in-vitro process. The second binding substance 19A was a DNA aptamer having a base sequence of SEQ ID NO: 3 with a biotinylation-modified 5' end. The second binding substance 19C was a DNA aptamer having a base sequence of SEQ ID NO: 4 with a biotinylation-modified 5' end.

The base sequence of the second binding substance 19A was "5'-GATATCAACCCATGGTAGGTATTGCTTGGTAGGGATAGTGGGCTTGATGTT-3' ". The second binding substance 19A is described in Angew. Chem. 2021, 133, 10367-10373 (hereafter cited as Reference 3).

According to the description in Reference 3, the second binding substance 19A is a DNA aptamer, whose target substance is not an RBD, in the spike protein of the novel coronavirus SARS-CoV-2.

The base sequence of the second binding substance 19C was "5'-GCTGGATGTCACCGGATTGTCGGACATCGGATTGTCTGAGTCATATGACACATC CAGC-3' ". The DNA aptamer is described in Reference 2.

According to the description in Reference 2, the DNA aptamer having the base sequence of SEQ ID NO: 4 is a DNA aptamer whose target material is a portion of the nucleocapsid protein of the novel coronavirus SARS-CoV-2, the portion being not an area to which the base sequence of SEQ ID NO: 2 binds.

The second binding substance 19A was dissolved in 1x PBS/T to produce the second binding substance 19A solution. Then, the second binding substance 19C was dissolved in 1x PBS/T to produce the second binding substance 19C solution.

Each of the concentrations of the second binding substance 19A in the second binding substance 19A solution and the second binding substance 19C in the second binding substance 19C solution was 1 µmol/l. The second binding substance 19A solution and the second binding substance 19C solution were heated at 95°C and then slowly cooled.

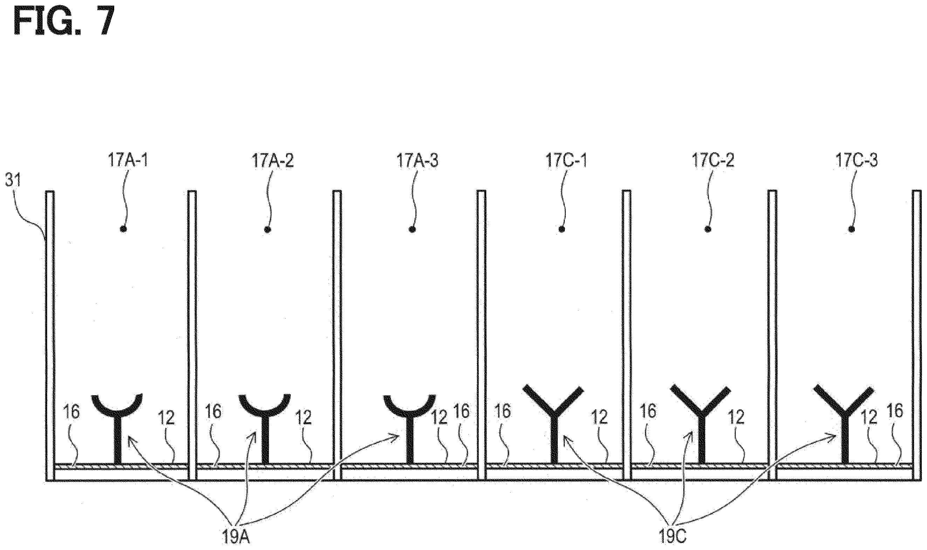

Next, in the process of S32 in FIG. 6, the avidin plate 31 (Sumitomo Bakelite Co., Ltd., product number BS-X7603) shown in FIG. 7 was prepared. The avidin plate 31 had a plurality of wells. Each well corresponds to a region. The inner surface 12 of each well is provided with avidin. The inner surface 12 corresponds to the base. The avidin is a chemical substituent 16 that modifies the DNA aptamer into the well through the interaction with biotin.

Then, 100 µL of the second binding substance 19A solution was placed in each of the three wells 17A-1, 17A-2, and 17A-3 included in the avidin plate 31 and allowed to stand for 1 hour at room temperature. At this time, the second binding substance 19A bound to the avidin provided on the inner surface 12 of the wells 17A-1, 17A-2, and 17A-3.

Meanwhile, 100 µL of the second binding substance 19C solution was placed in each of the three wells 17C-1, 17C-2, and 17C-3 included in the avidin plate 31 and allowed to stand for 1 hour at room temperature. At this time, the second binding substance 19C bound to the avidin provided on the inner surface 12 of the wells 17C-1, 17C-2, and 17C-3.

Next, the respective wells 17A-1, 17A-2, 17A-3, 17C-1, 17C-2, and 17C-3 were washed with 200 µL of 1x PBS/T. The washing was performed three times. At this time, the second binding substances 19A and 19C that did not bind to the avidin provided on the inner surface 12 of each well were removed.

Next, in a process of S33 in FIG. 6, biotin (Tokyo Chemical Industry Co., Ltd., product number B0463) was dissolved in 1x PBS/T to produce a biotin solution. The concentration of biotin in the biotin solution was 250 µmol/l.

Then, 200 µL of the biotin solution was placed in each of the wells 17A-1, 17A-2, 17A-3, 17C-1, 17C-2, and 17C-3 and allowed to stand for 2 hours at room temperature. At this time, biotin bound to the avidin which was provided on the inner surface 12 of each well and did not bind to the second binding substances 19A and 19C.

Next, the respective wells 17A-1, 17A-2, 17A-3, 17C-1, 17C-2, and 17C-3 were washed with 200 µL of 1x PBS/T. The washing was performed three times. At this time, biotin that did not bind to the wells was removed.

The target substances 7A and 7C to which the second binding substances 19A and 19B bind are different for each region. That is, the second binding substance 19A and the target substance 7A bind together in the wells 17A-1, 17A-2, and 17A-3. The second binding substance 19C and the target substance 7C bind together in the wells 17C-1, 17C-2, and 17C-3. The wells 17A-1, 17A-2, and 17A-3 and wells 17C-1, 17C-2, and 17C-3 correspond to the respective regions.

The second binding substances 19A and 19C may be binding substances other than DNA aptamers. The second binding substances 19A and 19C may be, for example, a low molecular weight protein preparation or an RNA aptamer. Examples of low molecule weight protein preparations include fragment antibodies, single chain antibodies, diabodies, nanobodies, VHHs, peptide aptamers, and the like.

The base sequences of the DNA aptamers constituting the second binding substances 19A and 19C may be base sequences other than those of SEQ ID NO: 1 and SEQ ID NO: 2. The base sequence of the DNA aptamer can be selected according to the target substance. When nucleic acid aptamers are used as the second binding substances 19A and 19C, a terminus other than the 5' end in the nucleic acid aptamer may bind to the base. When low molecular weight protein preparations are used as the second binding substances 19A and 19C, the N-terminus in the low molecular weight protein preparation may bind to the base, or the C-terminus may bind to the base.

The fixation of the second binding substances 19A and 19C to the base may be a binding other than a binding based on the biotin-streptavidin interaction. For example, a maleimide-coated plate (Tomsic Ltd., product part number MG22F-MAL) can be used as the container. The maleimide-coated plate includes maleimide on the inner surface 12 of each well. The inner surface 12 corresponds to the base. Maleimide corresponds to the chemical substituent 16. The inner surface 12 includes a bottom surface 12-1 and a first side surface 12-2. When the well faces the outer wall 51, the inner surface 12 further includes the second side surface 12-3. The maleimide included in the inner surface 12 binds to thiol contained in the second binding substances 19A and 19C, thereby fixing the second binding substances 19A and 19C to the inner surface 12. When the inner surface 12 of the container does not include the chemical substituent 16 or nucleic acid binding protein 18, the inner surface 12 can be provided with the chemical substituent 16 or nucleic acid binding protein 18 by a method of applying a material containing the chemical substituent 16 or nucleic acid binding protein 18 to the inner surface 12 or by a chemical modification method.

### (6-3) Implementation of analysis method for target substance

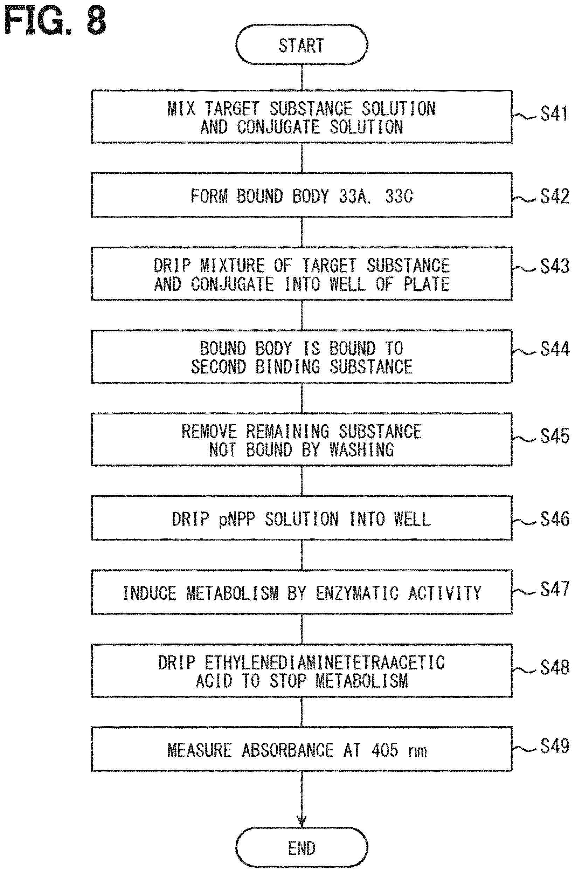

The analysis method for the target substance was implemented as shown in FIG. 8. In S41 of FIG. 8, the target substances 7A and 7C were prepared. The target substance 7A was SARS-CoV-2 (2019-nCoV) Spike S1-His Recombinant Protein (Sino Biological, Inc., product number 405 91-V08H). The amino acid sequence of the target substance 7A contains an RBD. The target substance 7A was dissolved in 1x PBS/T to produce a target substance 7A solution. The concentration of the target substance 7A in the target substance 7A solution was 4 µg/mL.

The target substance 7C was SARS-CoV-2 (2019-nCoV) Nucleocapsid-His Recombinant Protein (Sino Biological, Inc., product number 40588-V07E). The target substance 7C was dissolved in 1x PBS/T to produce a target substance 7C solution. The concentration of the target substance 7C in the target substance 7C solution was 4 µg/mL.

Then, 200 µL of the target substance 7A solution and 200 µL of the target substance 7C solution were mixed together to produce a target substance solution. The concentration of the target substance 7A in the target substance solution was 2 µg/mL, and the concentration of the target substance 7C was 2 µg/mL.

Then, 50 µL of the conjugate 1A solution, 50 µL of the conjugate 1C solution, and 100 µL of the target substance solution were mixed and allowed to stand at room temperature for 1 hour to produce a conjugate 1A1C-target substance mixture.

In the conjugate 1A1C-target substance mixture, the concentration of the conjugate 1A was 5 µmol/l, the concentration of the conjugate 1C was 5 µmol/l, the concentration of the target substance 7A was 1 µg/mL, and the concentration of the target substance 7C was 1 µg/mL.

Further, 50 µL of the conjugate 1A solution, 100 µL of the target substance solution, and 50 µL of 1x PBS/T were mixed together and allowed to stand at room temperature for 1 hour to thereby produce a conjugate 1A-target substance mixture. The concentration of the conjugate 1A in the conjugate 1A-target substance mixture was 5 µmol/l, the concentration of the target substance 7A was 1 µg/mL, and the concentration of the target substance 7C was 1 µg/mL.

Further, 50 µL of the conjugate 1C solution, 100 µL of the target substance solution, and 50 µL of 1x PBS/T were mixed together and allowed to stand at room temperature for 1 hour to thereby produce a conjugate 1C-target substance mixture. The concentration of the conjugate 1C in the conjugate 1C-target substance mixture was 5 µmol/l, the concentration of the target substance 7A was 1 µg/mL, and the concentration of the target substance 7C was 1 µg/mL.

At this time, as shown in S42 of FIG. 8, the target substance 7A and the conjugate 1A formed a bound body 33A. Therefore, the target substance 7C and the conjugate 1C formed a bound body 33C.

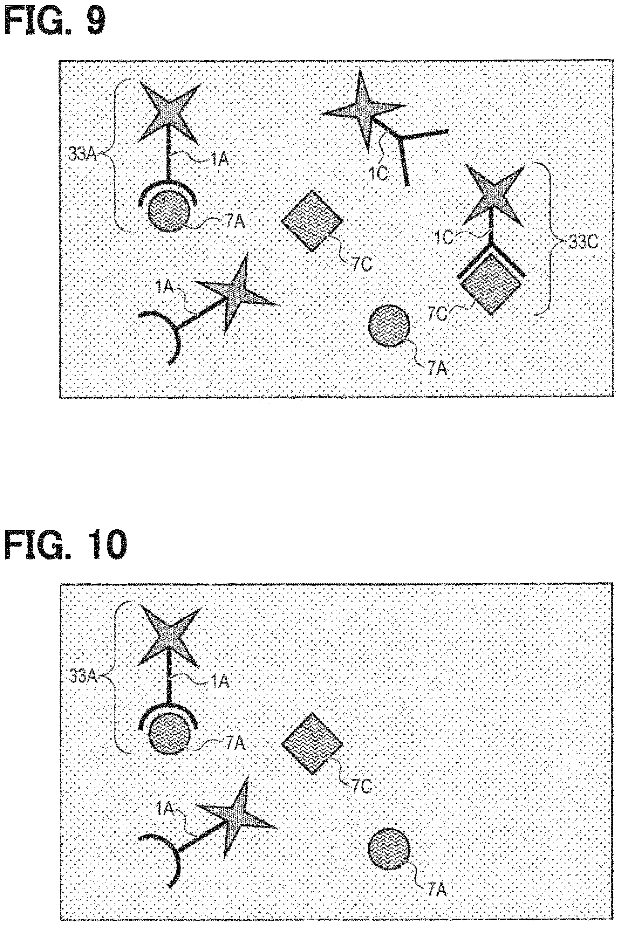

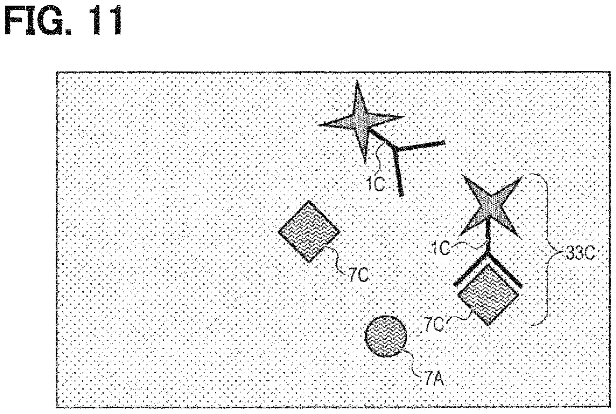

That is, as shown in FIG. 9, in the conjugate 1A1C-target substance mixture, the target substance 7A and the conjugate 1A formed the bound body 33A, while the target substance 7C and the conjugate 1C formed a bound body 33C. As shown in FIG. 10, in the conjugate 1A-target substance mixture, the target substance 7A and the conjugate 1A formed the bound body 33A. As shown in FIG. 11, in the conjugate 1C-target substance mixture, the target substance 7C and the conjugate 1C formed the bound body 33C.

Next, the mixture was then placed in the wells as shown in S43 of FIG. 8. That is, the conjugate 1A1C-target substance mixture was placed in each of the wells 17A-1 and 17C-1 and allowed to stand for 1 hour at room temperature. The conjugate 1A-target substance mixture was placed in each of the wells 17A-2 and 17C-2 and allowed to stand for 1 hour at room temperature. The conjugate 1C-target substance mixture was placed in each of the wells 17A-3 and 17C-3 and allowed to stand for 1 hour at room temperature.

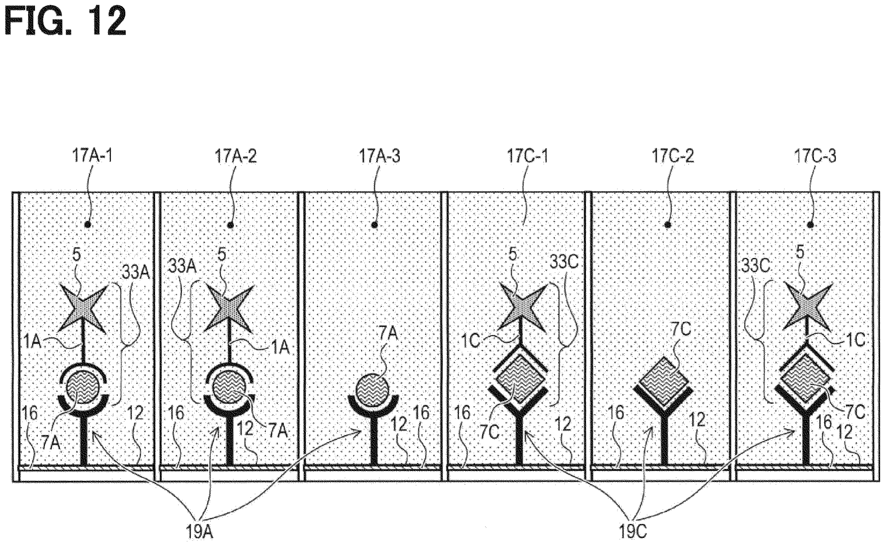

At this time, the bound bodies were bound to the second binding substances as shown in S44 of FIG. 8. In other words, as shown in FIG. 12, the bound body 33A was bound to the second binding substance 19A in the wells 17A-1 and 17A-2. Meanwhile, the bound body 33C was bound to the second binding substance 19C In the wells 17C-1 and 17C-3.

Next, the wells 17A-1, 17A-2, 17A-3, 17C-1, 17C-2, and 17C-3 were washed individually with 200 µL of 1x PBS/T, as shown in S45 of FIG. 8. The washing was performed three times. As a result, unbound substances were removed.

In the well 17A-1, other substances were removed except for the bound body 33A bound to the second binding substance 19A.

In the well 17A-2, other substances were removed except for the bound body 33A bound to the second binding substance 19A.

In the well 17A-3, other substances were removed except for the target substance 7A bound to the second binding substance 19A.

In the well 17C-1, other substances were removed except for the bound body 33C bound to the second binding substance 19C.

In the well 17C-2, other substances were removed except for the target substance 7C bound to the second binding substance 19C.

In the well 17C-3, other substances were removed except for the bound body 33C bound to the second binding substance 19C.

Next, in S46 of FIG. 8, 4-nitrophenyl phosphate disodium salt hexahydrate (Tokyo Chemical Industry Co., Ltd., product number N0241) was prepared. The 4-nitrophenyl phosphate disodium salt hexahydrate is hereafter referred to as pNPP. pNPP is a substrate for alkaline phosphatase-induced metabolism.

Next, pNPP was dissolved in a solution containing a carbonate pH standard solution (Fujifilm Wako Pure Chemicals, product no. 037-16145) and magnesium sulfate (Fujifilm Wako Pure Chemicals, product no. 137-12335) to produce a pNPP solution. The concentration of pNPP in the pNPP solution was 10 mmol/l. The solution containing the carbonate pH standard solution and magnesium sulfate contained 1 mmol/l of carbonate pH standard solution and 1 mmol/l of magnesium sulfate. The pH of the solution containing the carbonate pH standard solution and magnesium sulfate was adjusted to 9.6.

The solution in which pNPP was dissolved may be a solution other than the solution that contained the carbonate pH standard solution and magnesium sulfate. Examples of solutions in which pNPP was dissolved include a Tris buffer solution, a phosphate buffer solution, a Good's buffer solution, and the like. Examples of Good's buffer solutions include MES, Bis-Tris, ADA, PIPES, ACES, MOPSO, BES, MOPS, TES, HEPES, DIPSO, TAPSO, POPSO, HEPPSO, EPPS, Tricine, Bicine, TAPS, CHES, CAPSO, CAPS, and the like.

The pH of the solution in which the pNPP was dissolved may be a value other than 9.6. The solution in which pNPP is dissolved is preferably a weakly alkaline solution. The pH of the solution in which pNPP is dissolved is preferably 8 or higher and 11 or lower. When the solution in which pNPP is dissolved is a weakly alkaline solution, the change in pH derived from metabolism using pNPP as the substrate becomes significant. When the pH of the solution in which pNPP is dissolved is 8 or higher and 11 or lower, the change in pH derived from metabolism using pNPP as the substrate becomes significant.

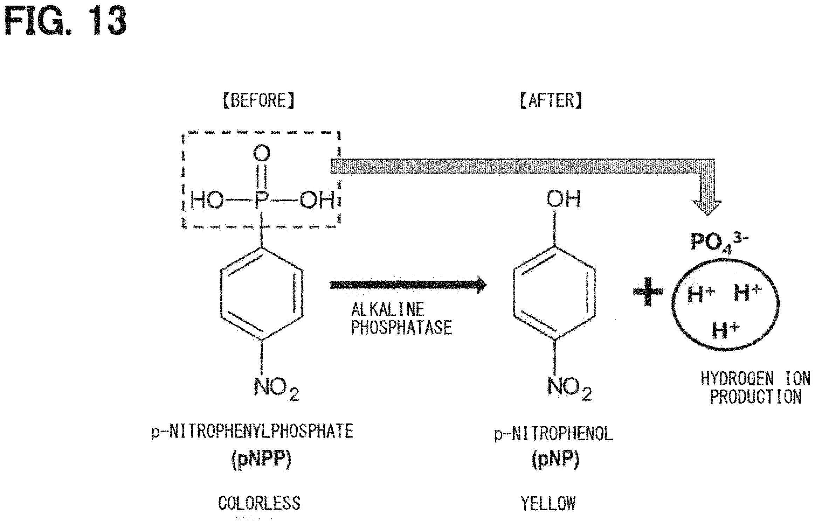

Then, as in S46 of FIG. 8, 100 µL of the pNPP solution was placed in each of the wells 17A-1, 17A-2, 17A-3, 17C-1, 17C-2, and 17C-3 and allowed to stand at 37°C for 10 minutes. At this time, as shown in S47 of FIG. 8, in the wells 17A-1, 17A-2, 17C-1, and 17C-3, metabolism using pNPP as the substrate was induced by the enzymatic activity of the labeled substance 5 included in the conjugate 1A or conjugate 1C, separating a phosphate group from the pNPP. The mode of the metabolism is shown in FIG. 13.

As a result, inorganic phosphoric acid and p-nitrophenol were produced in the wells 17A-1, 17A-2, 17C-1, and 17C-3. The absorption maximum wavelength of p-nitrophenol is 405 nm. Therefore, the solution containing p-nitrophenol turned yellow. The produced inorganic phosphoric acid ionized in the solution, releasing hydrogen ions. As a result, the pH of the solution decreased.

Then, as shown in S48 of FIG. 8, 5 µL of 0.5 mol/l ethylenediaminetetraacetic acid (FUJIFILM Wako Pure Chemical Corporation, product number 311-90075) was placed in each of the wells 17A-1, 17A-2, 17A-3, 17C-1, 17C-2, and 17C-3. At this time, metabolism using an alkaline phosphatase was stopped.

Next, as shown in S49 of FIG. 8, the absorbance of each of the wells 17A-1, 17A-2, 17A-3, 17C-1, 17C-2, and 17C-3 with light at a wavelength of 405 nm was measured using a microplate reader (Bio Tek Instruments, Inc., CYTATION3). The absorbance of the pNPP solution alone was also measured in the same manner.

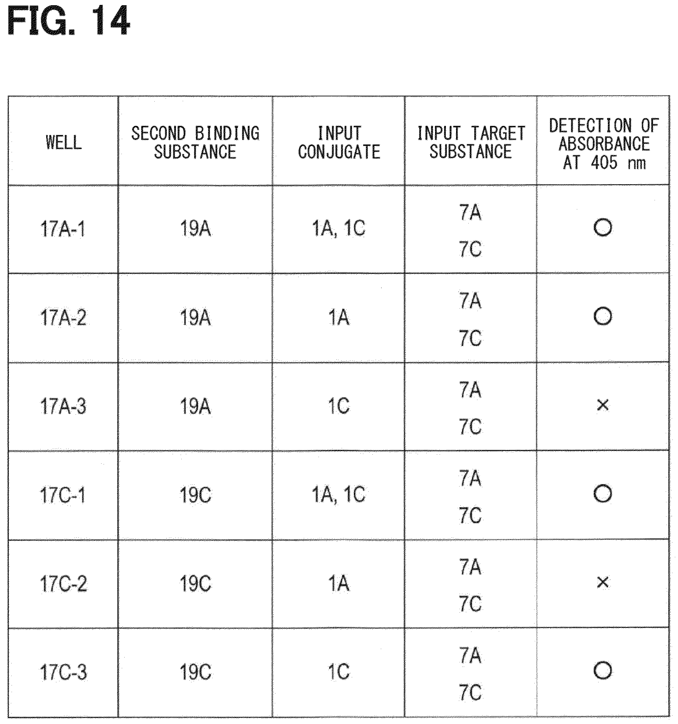

The absorbance measurement results are shown in FIG. 14. In FIG. 14, "o" means that the absorbance is greater than the absorbance observed when measuring only the pNPP solution. "X" means that the absorbance is the same or lower than the absorbance observed when measuring only the pNPP solution. Note that the enzymatic reaction catalyzed by alkaline phosphatase does not occur in a solution consisting of only the pNPP solution.

The absorbance measurements indicate the following. The greater absorbance is a phenomenon of the generation of the conjugate 1A bound to the target substance 7A, which is bound to the second binding substance 19A. The greater absorbance is a phenomenon of the generation of the conjugate 1C bound to the target substance 7C, which is bound to the second binding substance 19C.

In the wells 17A-1 and 17A-2, the second binding substance 19A is bound, and the second binding substance 19C is not bound. Thus, the greater absorbance in the wells 17A-1 and 17A-2 indicates that the mixture placed in the wells 17A-1 and 17A-2 contains the target substance 7A. Therefore, in Examples, the target substance 7A was able to be detected.

In the wells 17C-1 and 17C-3, the second binding substance 19C is bound, and the second binding substance 19A is not bound. Thus, the greater absorbance in the wells 17C-1 and 17C-3 indicates that the mixture placed in the wells 17C-1 and 17C-3 contains the target substance 7C. Therefore, in Examples, the target substance 7C was able to be detected.

The alkaline phosphatases included in the conjugates 1A and 1C also had enzymatic activity to induce metabolism using pNPP as the substrate, even when fused with the first binding substances 3A and 3C.

The first binding substance 3A included in the conjugate 1A also had binding activity to the target substance 7A even when fused with the labeled substance 5. The first binding substance 3C included in the conjugate 1C also had binding activity to the target substance 7C even when fused with the labeled substance 5.

The second binding substance 19A was also bound to the bound body 33A even in a state of being bound to the wells 17A-1 and 17A-2. Therefore, the second binding substance 19A had binding activity to the target substance 7A even in a state of being bound to the wells 17A-1 and 17A-2.

The second binding substance 19C was also bound to the bound body 33C even in a state of being bound to the wells 17C-1 and 17C-3. Therefore, the second binding substance 19C had binding activity to the target substance 7C even in a state of being bound to the wells 17C-1 and 17C-3.

The second binding substance 19A did not bind to the bound body 33C. Therefore, the second binding substance 19A did not have binding activity to the target substance 7C.

The second binding substance 19C did not bind to the bound body 33A. Therefore, the second binding substance 19C did not have binding activity to the target substance 7A.

### 7. Other Embodiments

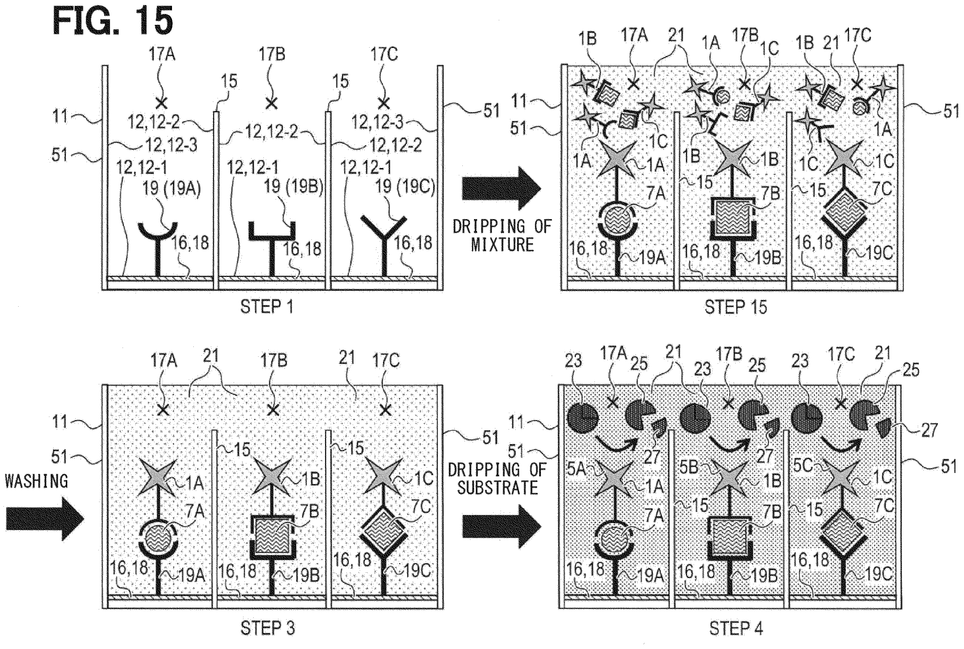

Although the embodiments of the present disclosure have been described above, the present disclosure is not limited to the embodiments described above, and can be implemented with various variations.
(1) As shown in FIG. 3, the base may be the surface 14 of the granular material 13. For example, the second binding substance 19 may be fixed to the surface 14 of the granular material 13, and the granular material 13 may be housed in the container 11. For example, the granular material 13A with the second binding substance 19A fixed thereto may be housed in the well 17A, the granular material 13B with the second binding substance 19B fixed thereto may be housed in the well 17B, and the granular material 13C with the second binding substance 19C fixed thereto may be housed in the well 17C. In this case, the effects of (1A) above can also be exhibited.
(2) The number of kinds of conjugates to be mixed with the sample is not particularly limited and can be, for example, 2, 3, 4, 5, 6, 7, 8.... The number of regions is not particularly limited and can be, for example, 2, 3, 4, 5, 6, 7, 8....
(3) The sample may be divided into portions and the analysis method of the present disclosure may be performed on a portion of the divided sample.
(4) As the container 11 used in the analysis method, the container 11 shown in FIG. 15 may be used instead of the container 11 shown in FIG. 2. In the case of the container 11 shown in FIG. 15, the tip of the partition wall 15 separating the wells 17A, 17B, and 17C is located closer to the bottom surface 12-1 of the container 11 than the tip of the outer wall 51 is. Therefore, a common region common to the respective wells 17A, 17B, and 17C is configured on an opening side of the container 1 separated from bottom surface 12-1 in the container 11. The respective wells 17A, 17B, and 17C can communicate with each other through this common region.
   Because of this configuration, when the mixture 21 is put into one of the wells 17A, 17B, or 17C up to the common region described above, the mixture 21 is also put into the other wells. When putting the mixture 21 into the wells 17A, 17B, and 17C, it is no longer necessary to divide the mixture 21 and individually put the mixture 21 into each of the wells 17A, 17B, and 17C. The mixture 21 can be put into each of the wells 17A, 17B, and 17C in a batch without dividing the mixture 21. This facilitates an operation of putting the mixture 21 into the wells 17A, 17B, and 17C.
(5) A plurality of functions associated with one component in the above embodiments may be implemented by a plurality of components, or one function associated with one component may be implemented by a plurality of components. A plurality of functions associated with a plurality of components may be implemented by a single component, or a single function implemented by a plurality of components may be implemented by a single component. Some of the configurations of the above embodiments may be omitted. Also, at least a portion of the configuration of the above embodiments may be added or substituted for other configurations of the above embodiments.
(6) In addition to the analysis methods described above, the present disclosure can also be realized in various forms, such as conjugates, groups consisting of a plurality of kinds of conjugates, producing methods for conjugates, and producing methods for the groups consisting of conjugates.

### [Technical ideas disclosed by the present specification]

### [Item 1]

An analysis method for target substances (7A, 7B, 7C), comprising:
mixing conjugates (1A, 1B, 1C) and a sample including the target substances to produce a mixture (21), the conjugates including first binding substances (3A, 3B, 3C), each having activity to bind to the corresponding target substance, and labeled substances (5A, 5B, 5C);
bringing the mixture into contact with second binding substances (19, 19A, 19B, 19C) fixed to a base (12, 14), each of the second binding substances having activity to bind to the corresponding target substance;
removing the target substance not bound to the second binding substance and the conjugate not bound to the target substance that is bound to the second binding substance; and
detecting a phenomenon caused by the labeled substance included in the conjugate bound to the target substance that is bound to the second binding substance, wherein
the first binding substance is a substance including a nucleic acid, or a substance including an amino acid,
the base is provided in a plurality of regions (17A, 17B, 17C), and the target substance to which the second binding substance binds is different for each region.

### [Item 2]

The analysis method according to Item 1, wherein
the region is composed of an inner surface (12) of a container (11), and
the base is the inner surface of the container or a surface (14) of a granular material (13).

### [Item 3]

The analysis method according to Item 1 or 2, wherein
the first binding substance is a nucleic acid aptamer or a low molecular weight protein preparation.

### [Item 4]

The analysis method according to any one of Items 1 to 3, wherein
the second binding substance is a protein, sugar, nucleic acid, low molecular weight compound, or lipid.

### [Item 5]

The analysis method according to any one of Items 1 to 4, wherein
at least a portion of the target substance is a protein, sugar, nucleic acid, low molecular weight compound, or lipid.

### [Item 6]

The analysis method according to any one of items 1 to 5, wherein
the labeled substance has at least one of an enzyme, a DNA zyme, an RNA zyme, magnetic labeling, fluorescent labeling, a chemiluminescent probe, or nanoparticles.

### [Item 7]

The analysis method according to Item 6, wherein
the phenomenon is production, consumption, or absorption of hydrogen ions, potassium ions, sodium ions, calcium ions, lithium ions, ammonium ions, or chloride ions, coloration, fluorescence, luminescence, phosphorescence, endotherm, exotherm, oxidation-reduction, or precipitation.

### [Item 8]

The analysis method according to any one of Items 1 to 7, wherein
each of the first binding substance and the labeled substance includes a chemical substituent or nucleic acid binding protein,
the labeled substance and the first binding substance are fused together by binding the chemical substituent or nucleic acid binding protein included in the labeled substance to the chemical substituent or nucleic acid binding protein included in the first binding substance, and
the chemical substituent has at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, or 5-halouracil.

### [Item 9]

The analysis method according to any one of Items 1 to 8, wherein
each of the second binding substance and the base includes a chemical substituent or nucleic acid binding protein,
the second binding substance is fixed to the base by binding the chemical substituent or nucleic acid binding protein included in the second binding substance to the chemical substituent or nucleic acid binding protein included in the base, and
the chemical substituent has at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, and 5-halouracil.

### [Item 10]

The analysis method according to any one of Items 1 to 9, wherein
the phenomenon is detected using a pH meter or ion-sensitive field effect transistor in a case where the phenomenon is production, consumption, or absorption of hydrogen ions, potassium ions, sodium ions, calcium ions, lithium ions, ammonium ions, or chloride ions,
the phenomenon is detected using a light-receiving device in a case where the phenomenon is coloration, luminescence, fluorescence, or phosphorescence,
the phenomenon is detected using a potentiometer in a case where the phenomenon is endotherm or exotherm,
the phenomenon is detected using a potentiometer or ion-sensitive field effect transistor in a case where the phenomenon is oxidation-reduction, and
the phenomenon is detected using a mass spectrometer, absorptiometer, or spectrophotometer in a case where the phenomenon is precipitation.

## Claims

1. An analysis method for a target substance (7A, 7B, 7C), comprising:
mixing a conjugate (1A, 1B, 1C) and a sample including the target substance to produce a mixture (21), the conjugate including a labeled substance (5A, 5B, 5C) and a first binding substance (3A, 3B, 3C) having activity to bind to the target substance;
bringing the mixture into contact with a second binding substance (19, 19A, 19B, 19C) fixed to a base (12, 14) and having activity to bind to the target substance;
removing the target substance not bound to the second binding substance and the conjugate not bound to the target substance that is bound to the second binding substance; and
detecting a phenomenon caused by the labeled substance included in the conjugate bound to the target substance that is bound to the second binding substance, wherein
the first binding substance is a substance including a nucleic acid, or a substance including an amino acid,
the base is provided in each of a plurality of regions (17A, 17B, 17C), and
the target substance to which the second binding substance binds is different among the plurality of regions.

2. The analysis method according to claim 1, wherein
the region is defined of an inner surface (12) of a container (11), and
the base is the inner surface of the container or a surface (14) of a granular material (13).

3. The analysis method according to claim 1 or 2, wherein
the first binding substance is a nucleic acid aptamer or a low molecular weight protein preparation.

4. The analysis method according to claim 1 or 2, wherein
the second binding substance is a protein, sugar, nucleic acid, low molecular weight compound, or lipid.

5. The analysis method according to claim 1 or 2, wherein
at least a portion of the target substance is a protein, sugar, nucleic acid, low molecular weight compound, or lipid.

6. The analysis method according to claim 1 or 2, wherein
the labeled substance has at least one of an enzyme, a DNA zyme, an RNA zyme, magnetic labeling, fluorescent labeling, a chemiluminescent probe, or nanoparticles.

7. The analysis method according to claim 6, wherein
the phenomenon is production, consumption, or absorption of hydrogen ions, potassium ions, sodium ions, calcium ions, lithium ions, ammonium ions, or chloride ions, coloration, fluorescence, luminescence, phosphorescence, endotherm, exotherm, oxidation-reduction, or precipitation.

8. The analysis method according to claim 1 or 2, wherein
each of the first binding substance and the labeled substance includes a chemical substituent or nucleic acid binding protein,
the labeled substance and the first binding substance are fused together by binding the chemical substituent or nucleic acid binding protein included in the labeled substance to the chemical substituent or nucleic acid binding protein included in the first binding substance, and
the chemical substituent has at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, or 5-halouracil.

9. The analysis method according to claim 1 or 2, wherein
each of the second binding substance and the base includes a chemical substituent or nucleic acid binding protein,
the second binding substance is fixed to the base by binding the chemical substituent or nucleic acid binding protein included in the second binding substance to the chemical substituent or nucleic acid binding protein included in the base, and
the chemical substituent has at least one of biotin, primary amine, azide, alkyne, dibenzocyclooctyne, bicyclononyne, 2'-O-propargyl, 2'-O-propargyl, thiol, avidin, streptavidin, neutravidin, N-hydroxysuccinimide, maleimide, and 5-halouracil.

10. The analysis method according to claim 1 or 2, wherein
the phenomenon is detected using a pH meter or ion-sensitive field effect transistor in a case where the phenomenon is production, consumption, or absorption of hydrogen ions, potassium ions, sodium ions, calcium ions, lithium ions, ammonium ions, or chloride ions,
the phenomenon is detected using a light-receiving device in a case where the phenomenon is coloration, luminescence, fluorescence, or phosphorescence,
the phenomenon is detected using a potentiometer in a case where the phenomenon is endotherm or exotherm,
the phenomenon is detected using a potentiometer or ion-sensitive field effect transistor in a case where the phenomenon is oxidation-reduction, and
the phenomenon is detected using a mass spectrometer, absorptiometer, or spectrophotometer in a case where the phenomenon is precipitation.
